# EUROPEAN PATENT SPECIFICATION

(11) **EP 4 047 369 B1**
(45) Date of publication and mention of the grant of the patent: **20.08.2025**
(21) Application number: 19949154.9
(22) Date of filing: 14.10.2019
(51) Int. Cl.: G01N 33/569, G01N 33/576, G01N 33/543, G01N 33/68, G01N 21/76, C07K 14/005, C12N 9/08, C12N 9/16

(54) **KIT AND METHOD FOR DETECTING HCV ANTIBODY**
KIT UND VERFAHREN ZUM NACHWEIS VON HCV-ANTIKÖRPER
KIT ET PROCÉDÉ DE DÉTECTION D'ANTICORPS CONTRE LE VHC

(43) Date of publication of application: 24.08.2022
(73) Proprietor: Shenzhen Mindray Bio-Medical Electronics Co., Ltd, Shenzhen, Guangdong 518057 (CN)
(72) Inventor: LI, Ke, Shenzhen, Guangdong 518057 (CN); JIANG, Ming, Shenzhen, Guangdong 518057 (CN); HE, Jianwen, Shenzhen, Guangdong 518057 (CN); ZHANG, Yuping, Shenzhen, Guangdong 518057 (CN)
(74) Representative: KIPA AB
(86) International application number: PCT/CN2019/111102
(87) International publication number: WO 2021/072607

(56) References cited:
- EP-A1- 4 016 080
- WO-A1-2016/207343
- CN-A- 1 755 365
- CN-A- 101 551 394
- CN-A- 104 697 988
- CN-A- 107 290 523
- CN-A- 109 187 954
- CN-B- 104 697 988
- US-B2- 8 865 398
- CAO CHENG ET AL, CHINESE JOURNAL OF LABORATORY MEDICINE, vol. 22, no. 6, 1 November 1999 (1999-11-01), XP055801320
- CAO CHENG, LI PING, SHI CHENGHUA: "HCV antibodies detection using recombinant β galactosidase HCV antigens chimerical protein as enzyme conjugate", CHINESE JOURNAL OF LABORATORY MEDICINE, vol. 22, no. 6, 1 November 1999 (1999-11-01), XP055801320

## Description

### TECHNICAL FIELD

Embodiments of the present application relate to the field of hepatitis C virus (HCV) antibody detection, and in particular, to enzyme label conjugate used in HCV antibody immunoassay, especially a double-antigen sandwich assay or an immunocapture assay.

### BACKGROUND

Hepatitis C is a blood-borne disease caused by hepatitis C virus (HCV). Chronic HCV infection may lead to chronic liver inflammation and necrosis and liver fibrosis, and some patients may develop liver cirrhosis or even hepatocellular carcinoma (HCC), which is extremely harmful to the health and life of patients and has become a serious social and public health problem. Hepatitis C is a global epidemic and is the leading cause of end-stage liver diseases in Europe, United States, Japan, etc. In the United States, the number of HCV-infected people is four times that of HIV-infected people. By 2010, more people died from HCV infection than from HIV infection. According to the World Health Organization, the global infection rate of HCV is about 3%, and it is estimated that about 170 million people are infected with HCV, and about 35,000 new hepatitis C cases are diagnosed each year.

At present, there is no effective vaccine against hepatitis C, so the control of hepatitis C can only begin with the control of the source of infection and the transmission route. Therefore, early and accurate diagnosis and detection of HCV infection is the most effective means to prevent and control the source of hepatitis C infection and block the transmission route, and immunoassay is one of the main methods for the diagnosis and management of hepatitis C virus.

HCV immunoassays include enzyme-linked immunosorbent assay, chemiluminescence immunoassay, gold-labelled antigen detection method, fluorescence immunoassay, *etc.* Among them, enzyme immunoassay techniques such as enzyme-linked immunosorbent assay and enzymatic chemiluminescence immunoassay are common HCV immunoassay methods using enzymes for labelling as reporter molecules. In reaction mode of the double-antigen sandwich assay, in the enzyme immunoassay, conjugate of enzyme for labelling and antigen substance, antibody to be detected in sample and antigen substance coated on solid phase support form a sandwich structure, and a qualitative or quantitative detection result of HCV is obtained by analyzing the enzyme for labelling in the sandwich structure. In reaction mode of the immunocapture assay, in the enzyme immunoassay, conjugate of enzyme for labelling and antigen substance, antibody to be detected in sample and anti-human IgG and anti-human IgM antibodies coated on solid phase support form a complex., a qualitative or quantitative detection result of HCV is obtained by analyzing the enzyme for labelling in the complex.

Generally, in the HCV enzyme immunoassay based on the double-antigen sandwich assay or the immunocapture assay, chemically activated cross-linking method is used to link the enzyme for labelling with the antigen substance to form a crosslinked compound or a conjugate. However, this link process has the disadvantages of being complicated to operate and difficult to control; in addition, such linking manner produce a non-uniform molecular molar ratio of the enzyme to the antigen, resulting in the product being a mixture of enzyme-antigen conjugates with different molecular molar ratio. Therefore, the application of the mixture of enzyme-antigen conjugates prepared by the chemical cross-linking method in the detection of hepatitis C virus antibody leads to problems such as difficult production control of hepatitis C detection kits and large batch-to-batch variation of test results.

Cao Cheng et al. (Chinese Journal of Laboratory Medicine, vol. 22, no. 6, 1 November 1999) discloses an ELISA system based on a sandwich method, comprising use of genetic engineering to express fusion protein of HCV chimerical antigens in E. coli.

CN 104697988B discloses a kit for detection of hepatitis c virus antibody and a method for the detection, using HCV fused antigens.

CN 1755365 discloses a fusion protein of chimeric HCV antigens HCV-C, NS3 and NS4 chemically connected with horseradish peroxidase.

US 8865398B2 discloses a kit comprising HCV antigen core peptides or NS3 peptide coupled by SMASA functional group to horseradish peroxidase.

### SUMMARY

In order to solve the problems above, embodiments of the present application provide an immunoassay kit for HCV antibody, including a fusion protein of an enzyme for labelling and an HCV antigenic protein. The present invention is defined by the appended claims.

As used in the embodiments of the present application, "antigenic protein" or "antigen substance" refers to a protein that is immunoreactive and can be used in HCV immunological detection; it can be one HCV antigen or a fragment thereof, or a fusion antigen of two or more HCV antigens or fragments thereof (or a chimeric protein of two or more HCV antigens or fragments thereof).

In the embodiments of this application, the antigenic protein contains one or more of HCV core antigen, HCV NS3 antigen, HCV NS4 antigen and HCV NS5 antigen, or the antigenic protein contains a fusion antigen of two or more of HCV core antigen, HCV NS3 antigen, HCV NS4 antigen and HCV NS5 antigen. Core, NS3, NS4, and NS5 are the structural and membrane proteins of hepatitis C virus. A large number of studies have shown that Core, NS3, NS4, and NS5 not only have high immunogenicity, but also can be used as hepatitis C virus diagnostic antigens.

As used in the embodiments of the present application, "HCV antigen" refers to a substance that has immunoreactivity and can be used in HCV immunoassay, and is selected from HCV conserved protein or a fragment thereof. Exemplary HCV antigen can be HCV core antigen, HCV NS3 antigen, HCV NS4 antigen or HCV NS5 antigen. In the embodiments of the present application, the HCV antigen may exist in the form of one or more copies.

In some embodiments, the HCV antigenic protein of the present application contains HCV core antigen. For example, the HCV antigenic protein of the present application contains HCV core antigen, HCV NS3 antigen, HCV NS4 antigen and HCV NS5 antigen.

In some embodiments, the antigenic protein is a chimeric protein or a fusion antigen containing HCV core antigen and one or more additional HCV antigens. Wherein, the additional HCV antigen may be a highly immunoreactive HCV antigen, for example, one or more of HCV NS3 antigen, HCV NS4 antigen and HCV NS5 antigen.

In an exemplary embodiment, the HCV antigenic protein of the present application is a chimeric protein containing HCV core antigen and HCV NS3, or a chimeric protein containing HCV core antigen, HCV NS3 antigen and HCV NS4, or a chimeric protein containing HCV core antigen, HCV NS3 antigen, HCV NS4 antigen and HCV NS5 antigen.

When the antigenic protein contains two or more HCV antigens, these HCV antigens may be present in any order. In an exemplary embodiment, the antigenic protein of the present application may contain HCV core antigen and HCV NS3 antigen in the order from N-terminus to C-terminus; alternatively, the antigenic protein of the present application may contain HCV NS3 antigen and HCV core antigen in the order from N-terminus to C-terminus.

In a specific embodiment, the HCV antigenic protein of the present application contains HCV core antigen, HCV NS3 antigen, HCV NS4 antigen and HCV NS5 antigen in the order from N-terminus to C-terminus.

In a specific embodiment, the antigenic protein of the present application contains HCV NS3 antigen, HCV NS4 antigen, HCV core antigen and HCV NS5 antigen in the order from N-terminus to C-terminus.

In a specific embodiment, the antigenic protein of the present application contains HCV NS3 antigen, HCV core antigen, HCV NS4 antigen and HCV NS5 antigen in the order from N-terminus to C-terminus.

In the embodiments of the present application, the HCV antigens may be directly linked with each other or may be linked through a Linker with each other, as long as the HCV antigens are linked while ensuring that their respective structures and activities are not affected. In the embodiments of the present application, a flexible linker may be used, such as flexible (Gly₄Ser)ₙ, GGGS, GGSGGGSG, and the like.

In the embodiments of the present application, "enzyme" and "enzyme for labelling" may be used interchangeably, and refer to an enzyme used in enzyme immunoassays. For example, it may be an enzyme used in enzyme-linked immunosorbent assay and enzymatic chemiluminescence immunoassay.

In the present invention, the enzyme is alkaline phosphatase (EC 3.1.3.1), which, for example, can catalyze the hydrolysis of phosphate group-containing chromogenic substrates and chemiluminescent substrates such as nitrophenyl phosphate (PNP), sodium β-glycerophosphate, naphthyl phosphate, 3-(2-spiroadamantane)-4-methoxy-4-(3-phosphoryloxy)-phenyl-1,2-dioxetane (AMPPD).

The alkaline phosphatase in the embodiments of the present application may be naturally occurring, artificially synthesized or produced by genetic engineering. In addition, the alkaline phosphatase in the embodiments of the present application may be a modified, such as surface glycosylated or deglycosylated alkaline phosphatase.

The source of alkaline phosphatase is not particularly limited in the embodiments of the present application, as long as the enzyme immunoassay can be realized. Exemplary alkaline phosphatase can be derived from but not limited to bacteria, such as *E. coli*; mammals such as cattle (for example Genebank: AF052227.1 (https://www.ncbi.nlm.nih.gov/)) or human (for example Genebank: M12551.1); and shrimp.

In another embodiment of the invention, the enzyme is horseradish peroxidase (EC 1.11.1.7), which uses iron porphyrin as a prosthetic group and can catalyze the polymerization of phenol, aniline and their substitutes in the presence of hydrogen peroxide, and is widely distributed in the plant kingdom, with the highest content in horseradish.

The horseradish peroxidase in the embodiments of the present application may be naturally occurring, artificially synthesized or produced by genetic engineering. In addition, the horseradish peroxidase in the embodiments of the present application may be modified.

In some embodiments, the enzyme of the present application also includes mutants thereof. Compared with wild type, the mutants of the enzymes in the embodiments of the present application have more than 80%, optionally more than 85%, more than 90%, more than 95%, more than 98% or more than 99% sequence homology. An exemplary alkaline phosphatase mutant may be GeneBank: M29670.1 (https://www.ncbi.nlm.nih.gov/), but the embodiments of the present application are not limited thereto. An exemplary horseradish peroxidase mutant may be GeneBank: XM_018585035.1, but the embodiments of the present application are not limited thereto.

Sequence homology is determined by comparing a test sequence to a reference sequence by using a wild-type sequence as the reference sequence. The sequence homology of the test sequence relative to the reference sequence is then calculated using a sequence comparison algorithm. Two examples of algorithms suitable for determining sequence homology are the BLAST and BLAST 2.0 algorithms, described in Altschul et al. (1977) Nuc. Acids Res. 25:3389-3402, and Altschul et al. (1990), J. Mol. Biol. 215:403-410, respectively. Software for performing BLAST analysis is publicly available through NCBI.

In the present invention, the enzyme is fused to any position of the antigenic protein. The enzyme may be fused to the N-terminus of the HCV antigenic protein; alternatively, the enzyme may be fused to the C-terminus of the antigenic protein; alternatively, the enzyme may be fused between any two adjacent antigens in the antigenic protein.

In some embodiments, the fusion protein of the HCV antigenic protein and the enzyme contains enzyme, HCV core antigen, HCV NS3 antigen, and HCV NS4 antigen in the order from N-terminus to C-terminus.

In some embodiments, the fusion protein of the HCV antigenic protein and the enzyme contains enzyme, HCV core antigen, HCV NS3 antigen, HCV NS4 antigen, and HCV NS5 antigen in the order from N-terminus to C-terminus.

In some embodiments, the fusion protein of the HCV antigenic protein and the enzyme contains HCV core antigen, HCV NS3 antigen, HCV NS4 antigen, HCV NS5 antigen and enzyme in the order from N-terminus to C-terminus.

In some embodiments, the fusion protein of the HCV antigenic protein and the enzyme contains HCV core antigen, HCV NS3 antigen, enzyme, HCV NS4 antigen, and HCV NS5 antigen in the order from N-terminus to C-terminus.

In a specific embodiment, the fusion protein of the present application contains, in the order from N-terminus to C-terminus, alkaline phosphatase, HCV core antigen, HCV NS3 antigen, HCV NS4 antigen and HCV NS5 antigen; or the fusion protein of the present application contains, in the order from N-terminus to C-terminus, HCV core antigen, HCV NS3 antigen, HCV NS4 antigen, HCV NS5 antigen and alkaline phosphatase; alternatively, the fusion protein of the present application contains, in the order from N-terminus to C-terminus, HCV core antigen, HCV NS3 antigen, alkaline phosphatase, HCV NS4 antigen and HCV NS5 antigen; alternatively, the fusion protein of the present application contains, in the order from N-terminus to C-terminus, horseradish peroxidase, HCV core antigen, HCV NS3 antigen, HCV NS4 antigen and HCV NS5 antigen; alternatively, the fusion protein of the present application contains, in the order from N-terminus to C-terminus, HCV core antigen, HCV NS3 antigen, HCV NS4 antigen, HCV NS5 antigen and horseradish peroxidase; alternatively, the fusion protein of the present application contains, in the order from N-terminus to C-terminus, HCV core antigen, HCV NS3 antigen, horseradish peroxidase, HCV NS4 antigen and HCV NS5 antigen.

In a specific embodiment, the immunoassay kit may include at least one fusion protein of: a fusion protein of alkaline phosphatase and HCV core antigen, a fusion protein of alkaline phosphatase and HCV NS3 antigen, a fusion protein of alkaline phosphatase and HCV NS4 antigen, and a fusion protein of alkaline phosphatase and HCV NS5 antigen; or the immunoassay kit may include at least one fusion protein of: a fusion protein of horseradish peroxidase and HCV core antigen, a fusion protein of horseradish peroxidase and HCV NS3 antigen, a fusion protein of horseradish peroxidase and HCV NS4 antigen, and a fusion protein of horseradish peroxidase and HCV NS5 antigen.

In the embodiments of the present application, the enzyme may be directly linked to the antigenic protein or may be linked to the antigenic protein through a Linker, as long as the enzyme and the antigenic protein are linked while ensuring that their respective structures and activities are not affected. In the embodiments of the present application, a flexible linker is used, such as flexible (Gly₄Ser)ₙ, GGGS, or GGSGGGSG.

A "fusion protein" refers to a fusion protein of an enzyme and an antigenic protein. For example, it may be expressed as enzyme + core + NS3, in this case, it refers to a fusion protein of enzyme, HCV core antigen and HCV NS3 antigen in the order from N-terminus to C-terminus.

The fusion protein of the enzyme and the HCV antigenic protein may be prepared by conventional recombinant expression technology. In the embodiments of the present application, the recombinant expression technology may be prokaryotic expression technology, such as *E. coli* expression technology, *etc.*; and eukaryotic expression technology, such as yeast expression technology, insect cell expression technology, *etc.*

Those skilled in the art would understand that the kit in the embodiments of the present application may further include other reagents or components for the detection of HCV antibody based on double antigen sandwich assay or immunocapture assay, for example, include a solid phase support coated with an HCV antigenic protein (the HCV antigenic protein coated on the solid phase support and the HCV antigenic protein in the fusion protein can bind to a same HCV antibody in a sample) or a solid phase support coated with anti-human IgM antibody and anti-human IgG antibody; a calibrator for drawing a standard curve; a quality control for controlling quality; a substrate solution for chemiluminescence reaction; and/or a wash buffer and a sample diluent, and the like.

In some embodiments, the kit includes a solid phase support coated with a fusion antigen containing HCV core antigen, HCV NS3 antigen, HCV NS4 antigen, and HCV NS5 antigen; alternatively, the kit includes a solid phase support coated with HCV core antigen, a solid phase support coated with HCV NS3 antigen, a solid phase support coated with HCV NS4 antigen, and a solid phase support coated with HCV NS5 antigen.

On the other hand, the embodiments of the present application (but not part of the present invention) also relate to the use of a fusion protein of an enzyme and an HCV antigenic protein in the manufacture of an immunoassay kit for detecting HCV. The immunoassay kit includes:
a first reagent containing a solid phase coating on which a first ligand is coated, and the first ligand can bind to an HCV antibody in a sample;
a second reagent containing an enzyme label conjugate, wherein the enzyme label conjugate is a second ligand fused with an enzyme, where the second ligand is an HCV antigenic protein and can bind to the HCV antibody to which the first ligand binds.

In a specific instance, HCV is detected based on double antigen sandwich assay or immunocapture assay.

In a variation of the disclosure, an immunoassay kit for detecting HCV is provided, and the kit includes:
a first reagent containing a solid phase coating, wherein the solid phase coating is a solid phase support coated with an HCV antigenic protein (first ligand);
a second reagent containing an enzyme label conjugate, wherein the enzyme label conjugate is an HCV antigenic protein (second ligand) fused with an enzyme, where the HCV antigenic protein coated on the solid phase support and the HCV antigenic protein in the fusion protein is capable of binding to a same HCV antibody in a sample; and
an instruction describing detection based on double antigen sandwich assay.

Those skilled in the art can understand that the HCV antigenic protein coated on the solid phase support in the first reagent and the HCV antigenic protein fused in the fusion protein in the second reagent are of the same type, but the source or the linking manner or sequence between multiple antigens may be the same or different. For example, the HCV antigenic protein coated on the solid phase support may be a fusion antigen of HCV core antigen, HCV NS3 antigen, HCV NS4 antigen and HCV NS5 antigen linked in order, and the HCV antigenic protein fused in the fusion protein in the second reagent may be a fusion antigen of HCV core antigen, HCV NS4 antigen, HCV NS5 antigen and HCV NS3 antigen linked in order.

In addition, in the embodiments of the present application, the HCV antigenic protein coated on the solid phase support may be the HCV antigenic protein described above, or may be an HCV antigenic protein linked in other ways, such as linked chemically.

In the embodiments of the present application, an HCV antibody refers to an antibody produced in a subject after HCV infection, such as anti-HCV IgG antibody and anti-HCV IgM antibody.

In another variation of the embodiments of the present application, an immunoassay kit for detecting HCV is provided, and the kit includes:
a first reagent containing a solid phase coating, wherein the solid phase coating is a solid phase support coated with anti-human IgM antibody and anti-human IgG antibody (first ligand);
a second reagent containing an enzyme label conjugate, wherein the enzyme label conjugate is a fusion protein of an enzyme and an HCV antigenic protein (second ligand); and
an instruction describing detection based on immunocapture assay.

As used in the embodiments of the present application, "solid phase support" refers to a solid surface to which an antigen or an antibody can be attached. There is no particular limitation on the solid phase support used in the embodiments of the present application, and commercial solid phase supports and any solid phase supports that can be used in immunoassay can be used in the present application. Exemplary solid phase supports may be magnetic beads (such as superparamagnetic microspheres), ELISA plates, plastic plates, plastic tubes, latex beads, agarose beads, glass, nitrocellulose membranes, nylon membranes, silica plates or microchips, but the embodiments of the present application are not limited thereto.

In the embodiments of the present application, the solid phase coating may be contained in a conventional diluent containing proteins and surfactants and having buffer capacity.

In the embodiments of the present application, the enzyme label conjugate may be contained in a conventional diluent containing a protein and a surfactant and having buffer capacity.

In the embodiments of the present application, the fusion protein may be contained in the second reagent, for example, at a concentration of about 50 ng/mL to 150 ng/mL.

In a specific embodiment, the kit of the present application may further include a third reagent, which contains a blocking agent and a surfactant. For example, the blocking agent includes one or more components selected from the group consisting of: skimmed milk powder, BSA, gelatin, serum, casein, ovalbumin, animal IgG, a surfactant (for example: Tween-20, Tween-80, TritonX-100, *etc.*)*.*

In a specific embodiment, the kit of the present application may further include a fourth reagent, which contains a reducing agent. For example, the reducing agent includes one or more components selected from the group consisting of: DTT and β-mercaptoethanol.

In the embodiments of the present application, the blocking agent and the surfactant may be dissolved in a conventional diluent with buffering capacity; the reducing agent may be dissolved in a conventional diluent with buffering capacity.

In some embodiments, the kit may further include a reaction substrate for the enzyme for labelling, preferably the reaction substrate is 3-(2-spiroadamantane)-4-methoxy-4-(3-phosphoryloxy)-phenyl-1,2-dioxetane.

Unless otherwise specified, the terms "first", "second", "third" and "fourth" in the embodiments of the present application are only used to distinguish multiple similar elements, and not intended to indicate any difference in importance or order between the elements.

The present invention further relates to a method for detecting an HCV antibody produced after infection with HCV in a sample by using a fusion protein of an enzyme for labelling and a HCV antigenic protein, and the method includes the following steps:
mixing the sample and a solid phase support coated with a first ligand which is HCV antigenic protein, or anti-human IgG antibody and anti-human IgM antibody, so that the first ligand coated on the solid phase support is sufficiently bound to the HCV antibody in the sample;
washing the above mixture to remove unbound substances;
adding an enzyme label conjugate with a second ligand to the above washed mixture for mixing in which the enzyme label conjugate with a second ligand is the fusion protein of an enzyme for labelling and an HCV antigenic protein defined in claim 1 and the HCV antigenic protein is the second ligand, so that the second ligand in the enzyme label conjugate binds to the HCV antibody bound on the solid phase support to form a sandwich complex, wherein the enzyme label conjugate is a fusion protein of an enzyme for labelling and an HCV antigenic protein;
washing the sandwich complex above to remove unbound substances;
adding a chemiluminescent substrate to the above washed sandwich complex, and detecting a number of photons generated by the reaction to obtain a chemiluminescent signal value of the sample.

The first ligand is HCV antigenic protein, or anti-human IgG antibody and anti-human IgM antibody.

In the embodiments of the present application, a fusion protein of an enzyme for labelling and an HCV antigenic protein is obtained by recombinant expression, and the fusion protein can be applied to an HCV immunoassay kit based on double-antigen sandwich assay or immunocapture assay. It should be noted that:
by replacing conjugate of enzyme and antigen substance produced by chemically activated cross-linking with such a fusion protein, the fusion protein of the enzyme for labelling and the HCV antigenic protein in the kit has a uniform molar ratio of the enzyme for labelling to the HCV antigenic protein, thereby avoiding the problem of large batch-to-batch differences in kit production and test results.

On the other hand, compared with a conventional kit for double antigen sandwich assay or immunocapture assay, the kit in the embodiments of the present application shows better discrimination between negative and positive samples, and has a better sample coincidence rate.

On the other hand, the kit including the fusion protein in the embodiments of the present application avoids the destruction of the activity of the antigen or the activity of the enzyme caused by chemically activated cross-linking method.

On the other hand, the embodiments of the present application omit the step of chemical cross-linking reaction, which makes operation more convenient and faster.

### DESCRIPTION OF THE PREFERRED EMBODIMENTS

The technical solutions of the embodiments of the present application will be described below clearly and comprehensively.

As mentioned above, the fusion protein of the enzyme and the HCV antigenic protein in the embodiments of the present application may be produced by recombinant expression technology.

An exemplary recombinant expression method may include the following steps:
i) transfecting or transforming a nucleic acid molecule or an expression vector into a host cell;
ii) culturing the host cell under conditions of expressing a fusion protein; and
iii) isolating the fusion protein.

In the steps above, the nucleic acid molecule encodes the fusion protein above; the expression vector is used to prepare the fusion protein of the enzyme and the HCV antigenic protein by recombinant expression, the expression vector includes a nucleic acid molecule encoding the fusion protein above and operably linked to an expression control sequence, and additionally includes genetic elements for maintenance and propagation in the respective host cells, such as origins of replication and/or selectable marker genes.

### Preparation of the fusion protein

Production of the fusion protein using an *E. coli* expression system:
Step 1: confirmation of the gene of interest. According to the gene sequence and amino acid sequence of two proteins to be fused, a suitable linker is added between the two. For example, the gene sequence of the linker is GGA GGA GGA GGA TCA GGA GGA GGA GGA TCA GGA GGA GGA GGA TCA, and its corresponding amino acid sequence is protein sequence (Gly₄Ser)₃, and a His tag is added to C-terminus of the fusion protein for purification, and the sequence of the gene of interest is preliminarily determined. Then, through codon optimization, the final sequence of the gene of interest suitable for *E. coli* expression strain BL21 (DE3) is determined.
Step 2: vector construction. A suitable enzyme restriction site is selected and the gene of interest is integrated into a suitable expression vector pET28a.
Step 3: transformation and screening of positive cloned strains. The expression vector including the gene of interest is transformed into *E. coli* by electrotransformation or heat shock transformation. According to the reporter gene on the expression vector, an appropriate antibiotic is added to screen out positive strains.
Step 4: Expression and purification of protein.

Nucleic acid sequence information of HCV antigen: Core, GeneBank; AJ222676.1; NS3, GeneBank: EU84764.1; NS4, GeneBank: Z84363.1; NS5, GeneBank: S70341.1 (https://www.ncbi.nlm.nih.gov/).

### Reagent preparation method

### First reagent Ra:

3.5 mL of "magnetic beads coated with Core+NS3+NS4+NS5 chimeric protein" was measured off using a pipette or graduated cylinder and added to a magnetic bead-coated tube to replace a supernatant, that is, after magnetic separation was completed, the supernatant suctioned off and then an equal volume (3.5 mL) of a magnetic bead coating diluent was added and mixed evenly; the magnetic beads were mixed evenly and then added to a liquid preparation bottle containing 96.5 mL of the magnetic bead coating diluent; and the magnetic bead suspension was stirred until completely mixed to prepare the first reagent Ra, where the concentration of the magnetic beads coated with the Core+NS3+NS4+NS5 chimeric protein is 0.7 mg/mL; and the magnetic bead coating diluent is a conventional diluent with buffering capacity and contains a protein and a surfactant.

### Second reagent Rb:

99 mL of a tracer diluent is measured off using a suitable graduated cylinder and added to a liquid preparation bottle, 1 mL of "a fusion protein of an enzyme and an HCV antigenic protein" or "a conjugate of an enzyme and an HCV antigenic protein linked chemically" or "a conjugate of anti-human IgG and anti-human IgM linked chemically" was pipetted as an enzyme label conjugate using a pipettor and added into the tracer diluent; the solution was stirred with a stirrer to fully dissolve and mix evenly; then the prepared solution was filtered using a suitable filter with a pore size of 0.22 µm, and the filtrate was collected, to prepare the tracer Rb, where the concentration of the above enzyme label conjugate is 100 ng/mL; and the tracer diluent is a conventional diluent with buffering capacity and contains a protein and a surfactant.

Third reagent Rc:
a diluent with buffering capacity and containing BSA, Tween-20 and skimmed milk powder.

Fourth reagent Rd:
a diluent with buffering capacity and containing DDT.

### Double-antigen sandwich assay method

Step 1: the sample, the fourth reagent and the first reagent were added to a reaction tube and incubated at 37°C for 10 minutes, so that the HCV antigen coated on the magnetic bead solid phase were fully bound to anti-HCV IgG and anti-HCV IgM antibodies in the sample; and after incubation was complete, the magnetic bead solid phase was placed in a magnetic field and attracted, the substances bound to the magnetic bead solid phase were retained, and other unbound substances were washed off.

Step 2: the third reagent and the second reagent were added to the reaction tube and mixed evenly; After incubation at 37°C for 10 minutes, the HCV antigen on the enzyme label conjugate was bound to the anti-HCV IgG and anti-HCV IgM antibodies captured on the magnetic beads to form a sandwich complex. After incubation in the reaction tube was complete, the complex was attracted by the magnetic field, and other unbound substances were washed off.

Step 3: a chemiluminescent substrate was added to the reaction tube to generate chemiluminescence; and the number of photons produced by the reaction was then measured by a photomultiplier to obtain a chemiluminescence signal value of the sample.

In the embodiments of the present application, COI (Cutoff index) is the ratio of the chemiluminescence signal value (RLU) of the measured sample to a threshold value (Cutoff value), where COI ≥ 1 indicates that the measured sample is a positive sample, and COI < 1 indicates that the measured sample is a negative sample. For qualitative detection methods, the threshold value (cutoff value) is a cut-off value for judging whether the test result is positive or negative.

In the embodiments of the present application, the negative coincidence rate refers to the ratio of the number of samples determined to be negative using the test method according the embodiments of the present application to the negative samples actually involving in the evaluation, and the positive coincidence rate refers to the ratio of the number of samples determined to be positive using the test method according to the embodiments of the present application to the positive samples actually involving in the evaluation; the true negative and positive results of the samples came from hospital diagnoses.

### Example 1

In order to compare the HCV detection effect of a fusion protein of an enzyme and an HCV antigenic protein in the embodiments of the present application relative to that of a conjugate of the enzyme and the HCV antigenic protein produced by chemical cross-linking, the following three reagent combinations were prepared.

Combination 1: utilizing the *E. coli* expression system described above, a Core+NS3+NS4+NS5 chimeric protein (or fusion antigen) was used as an HCV antigenic protein to fuse with alkaline phosphatase to prepare the second reagent Rb, where in the fusion protein of the second reagent Rb, alkaline phosphatase (Genebank: AF052227.1 (https://www.ncbi.nlm.nih.gov/)) was fused at N-terminus of the Core+NS3+NS4+NS5 chimeric protein, and the Combination 1 was prepared according to the above "Reagent preparation method".

Combination 2: the second reagent Rb was prepared using a conjugate of alkaline phosphatase and Core+NS3+NS4+NS5 chimeric protein linked chemically, and the rest was the same as combination 1.

Next, 500 confirmed HCV negative samples and 100 confirmed positive samples from general hospitals were tested using combinations 1 and 2 according to the "Double-antigen sandwich assay method" described above, respectively. The results are shown in Table 1 below.

**Table 1**

| **Reagent combination** | **Combination 1** | **Combination 2** | **Combination 3 (indirect assay)** |
|---|---|---|---|
| Negative Sample 1 (COI) | 0.11 | 0.12 | 0.11 |
| Negative Sample 2 (COI) | 0.16 | 0.18 | 0.31 |
| Positive Sample 1 (COI) | 2.78 | 1.71 | 2.45 |
| Positive Sample 2 (COI) | 13.83 | 8.05 | 10.26 |
| Positive Sample 3 (COI) | 85.50 | 27.48 | 45.73 |
| Negative coincidence rate (500 samples) | 100% | 99.6% | 99% |
| Positive coincidence rate (100 samples) | 100% | 99% | 100% |

It can be seen from Table 1 that the negative coincidence rate of combination 1 is 100% and the positive coincidence rate is 100%, which is higher than the conventional double antigen sandwich assay using chemical linking (combination 2: negative coincidence rate is 99.6%, positive coincidence rate is 99%), and this result shows that: using the fusion protein of alkaline phosphatase and Core+NS3+NS4+NS5 can avoid the destruction of antigen activity or alkaline phosphatase activity caused by the preparation process using chemical linking method in the conventional double antigen sandwich assay. In the embodiments of the present application, both the alkaline phosphatase and the antigen in the fusion protein of alkaline phosphatase and Core+NS3+NS4+NS5 maintain high activity.

In addition, in order to prove the activity of the Core+NS3+NS4+NS5 chimeric protein used in the embodiments of the present application, reagent combination 3 was prepared: the second reagent Rb was prepared using a conjugate of alkaline phosphatase and anti-human IgG and anti-human IgM linked chemically, and the rest was the same as combination 1.

According to the indirect assay method, the 500 confirmed HCV negative samples and the 100 confirmed positive samples above were tested using the combination 3 above. The specific steps of the "indirect assay method" were as follows:
Step 1: the sample, the fourth reagent and the first reagent were added to a reaction tube and incubated at 37°C for 10 minutes, so that the HCV antigen coated on the magnetic bead solid phase were fully bound to anti-HCV IgG and anti-HCV IgM antibodies in the sample; and after incubation was complete, the magnetic bead solid phase was placed in a magnetic field and attracted, the substances bound to the magnetic bead solid phase were retained, and other unbound substances were washed off.
Step 2: the third reagent and the second reagent were added to the reaction tube and mixed evenly; After incubation at 37°C for 10 minutes, the anti-human IgG and anti-human IgM secondary antibodies on the enzyme label conjugate were bound to the anti-HCV IgG and anti-HCV IgM antibodies captured on the magnetic beads to form a sandwich complex. After incubation in the reaction tube was complete, the complex was attracted by the magnetic field, and other unbound substances were washed off.
Step 3: a chemiluminescent substrate was added to the reaction tube to generate chemiluminescence; and the number of photons produced by the reaction was then measured by a photomultiplier to obtain a chemiluminescence signal value of the sample.

The results are shown in Table 1. The indirect assay (combination 3) can better distinguish the negative samples from the positive samples, indicating that the Core+NS3+NS4+NS5 chimeric protein used in the embodiments of the present application has a good activity.

### Example 2

In order to compare the HCV detection effect of the fusion protein of the enzyme and the antigenic protein in the embodiments of the present application relative to that of the conjugate of the enzyme and the HCV antigenic protein produced by chemical cross-linking, the following three reagent combinations were prepared:
Combination 4: the *E. coli* expression system described above was used, Core+NS3+NS4+NS5 was used as an antigenic protein to fuse with horseradish peroxidase to prepare the second reagent Rb, wherein in the fusion protein of the second reagent Rb, horseradish peroxidase (GeneBank: KU504630.1 (https://www.ncbi.nlm.nih.gov/)) was located at N-terminus of the antigenic protein, and the Combination 4 was prepared according to "Reagent preparation method" above.
Combination 5: the second reagent Rb was prepared using a conjugate of horseradish peroxidase and Core+NS3+NS4+NS5 chimeric protein linked chemically, and the rest was the same as combination 4.

Next, the 500 HCV negative samples and the 100 positive samples in Example 1 were tested using combinations 4 and 5 according to the "Double antigen sandwich assay method" described above, respectively. The results are shown in Table 2 below.

**Table 2**

| **Reagent combination** | **Combination 4** | **Combination 5** | **Combination 6 (indirect assay)** |
|---|---|---|---|
| Negative Sample 1 (COI) | 0.21 | 0.19 | 0.42 |
| Negative Sample 2 (COI) | 0.26 | 0.25 | 0.51 |
| Positive Sample 1 (COI) | 1.53 | 1.24 | 1.39 |
| Positive Sample 2 (COI) | 4.21 | 2.97 | 3.78 |
| Positive Sample 3 (COI) | 25.44 | 15.04 | 24.36 |
| Negative coincidence rate (500 samples) | 100% | 99.4% | 99% |
| Positive coincidence rate (100 samples) | 100% | 98% | 100% |

It can be seen from Table 2 that the negative coincidence rate of combination 4 is 100% and the positive coincidence rate is 100%, which is higher than the conventional double antigen sandwich assay using chemical linking (combination 5: negative coincidence rate is 99.4%, positive coincidence rate is 98%), and this result shows that: using the fusion protein of horseradish peroxidase and Core+NS3+NS4+NS5 can avoid the destruction of antigen activity or horseradish peroxidase activity caused by the preparation process using chemical linking method in the conventional double antigen sandwich assay. In the embodiments of the present application, both the horseradish peroxidase and the antigen in the fusion protein of horseradish peroxidase and Core+NS3+NS4+NS5 maintain high activity.

Likewise, in order to prove the activity of the Core+NS3+NS4+NS5 chimeric protein used in the embodiments of the present application, reagent combination 6 was prepared: the second reagent Rb was prepared using a conjugate of horseradish peroxidase and anti-human IgG and anti-human IgM linked chemically, and the rest was the same as combination 4.

According to the indirect assay method above, the 500 confirmed HCV negative samples and the 100 confirmed positive samples above were tested using the combination 6 above. The results are shown in Table 1. The indirect assay (combination 6) can better distinguish the negative samples from the positive samples, indicating that the Core+NS3+NS4+NS5 chimeric protein used in the embodiments of the present application has a good activity.

### Example 3

In order to investigate the influence of the fusion position of the enzyme on the fusion protein in the embodiments of the present application, the following three reagent combinations were prepared:
Combination 1: the *E. coli* expression system described above was used, a Core+NS3+NS4+NS5 chimeric protein was used as an antigenic protein to fuse with alkaline phosphatase to prepare the second reagent Rb, where in the fusion protein of the second reagent Rb, alkaline phosphatase was fused at N-terminus of the Core+NS3+NS4+NS5 chimeric protein, and the Combination 1 was prepared according to "Reagent preparation method" above.
Combination 7: In the fusion protein of the second reagent Rb, alkaline phosphatase was fused at C-terminus of the Core+NS3+NS4+NS5 chimeric protein, and the rest was the same as combination 1.
Combination 8: In the fusion protein of the second reagent Rb, alkaline phosphatase was fused between NS3 and NS4 in the Core+NS3+NS4+NS5 chimeric protein, and the rest was the same as combination 1.

Next, the 500 confirmed HCV negative samples and the 100 confirmed positive samples from Example 1 were tested using combinations 1, 7 and 8 according to the "Double antigen sandwich assay method" described above, respectively, and the results are shown in Table 3 below.

**Table 3**

| **Reagent combination** | **Combination 1** | **Combination 7** | **Combination 8** |
|---|---|---|---|
| Negative Sample 1 (COI) | 0.11 | 0.10 | 0.12 |
| Negative Sample 2 (COI) | 0.16 | 0.17 | 0.15 |
| Positive Sample 1 (COI) | 2.78 | 2.92 | 2.65 |
| Positive Sample 2 (COI) | 13.83 | 14.32 | 13.17 |
| Positive Sample 3 (COI) | 85.50 | 89.78 | 82.43 |
| Negative coincidence rate (500 samples) | 100% | 100% | 100% |
| Positive coincidence rate (100 samples) | 100% | 100% | 100% |

It can be seen from Table 3 that when alkaline phosphatase is fused at N-terminus (combination 1), C-terminus (combination 7) and middle (combination 8) of the antigenic protein, both of the negative coincidence rate and the positive coincidence rate of the samples can reach 100%. Furthermore, while not wishing to be bound by theory, the inventors believe that, in the case of fusing the enzyme in the middle of the HCV antigenic protein, the active center of the enzyme would be partially blocked by other proteins, so fusion of the enzyme in the middle of the HCV antigenic protein is slightly less effective than fusion of the enzyme at N-terminus and C-terminus of the HCV antigenic protein.

### Example 4

In order to investigate the influence of the fusion position of the enzyme on the fusion protein in the embodiments of the present application, the following three reagent combinations were prepared:
Combination 4: the *E. coli* expression system described above was used, a Core+NS3+NS4+NS5 chimeric protein was used as an antigenic protein to fuse with horseradish peroxidase to prepare the second reagent Rb, where in the fusion protein of the second reagent Rb, horseradish peroxidase was fused at N-terminus of the Core+NS3+NS4+NS5 chimeric protein, and the Combination 4 was prepared according to "Reagent preparation method" above.
Combination 9: In the fusion protein of the second reagent Rb, horseradish peroxidase was fused at C-terminus of the Core+NS3+NS4+NS5 chimeric protein, and the rest was the same as combination 4.
Combination 10: In the fusion protein of the second reagent Rb, horseradish peroxidase was fused between NS3 and NS4 in the Core+NS3+NS4+NS5 chimeric protein, and the rest was the same as combination 4.

Next, the 500 confirmed HCV negative samples and the 100 confirmed positive samples from Example 1 were tested using combinations 4, 9 and 10 according to the "Double antigen sandwich assay method" described above, respectively, and the results are shown in Table 4 below.

**Table 4**

| **Reagent combination** | **Combination 4** | **Combination 9** | **Combination 10** |
|---|---|---|---|
| Negative Sample 1 (COI) | 0.21 | 0.24 | 0.22 |
| Negative Sample 2 (COI) | 0.26 | 0.29 | 0.27 |
| Positive Sample 1 (COI) | 1.53 | 1.95 | 1.42 |
| Positive Sample 2 (COI) | 4.21 | 4.50 | 3.93 |
| Positive Sample 3 (COI) | 25.44 | 24.72 | 23.22 |
| Negative coincidence rate (500 samples) | 100% | 100% | 100% |
| Positive coincidence rate (100 samples) | 100% | 100% | 100% |

It can be seen from Table 4 that when horseradish peroxidase is fused at N-terminus (combination 4), C-terminus (combination 9) and middle (combination 10) of the antigenic protein, both of the negative coincidence rate and the positive coincidence rate of the samples can reach 100%. Furthermore, while not wishing to be bound by theory, the inventors believe that, in the case of fusing the enzyme in the middle of the HCV antigenic protein, the active center of the enzyme would be partially blocked by other proteins, so fusion of the enzyme in the middle of the HCV antigenic protein is slightly less effective than fusion of the enzyme at N-terminus and C-terminus of the HCV antigenic protein.

### Example 5

In order to investigate the influence of the order of the antigenic proteins on the fusion protein in the embodiments of the present application, the following three reagent combinations were prepared:
Combination 1: combination 1 was prepared according to "Reagent preparation method" above, where the antigenic protein was Core+NS3+NS4+NS5 in the order from N-terminus to the C-terminus, and the alkaline phosphatase in the fusion protein was fused at N-terminus of the antigenic protein.
Combination 11: the antigenic protein was NS3+NS4+core+NS5 in the order from N-terminus to the C-terminus, and the rest was the same as combination 1.
Combination 12: the antigenic protein was NS3+core+NS4+NS5 in the order from N-terminus to the C-terminus, and the rest was the same as combination 1.

Next, the 500 confirmed HCV negative samples and the 100 confirmed positive samples from Example 1 were tested using combinations 1, 11 and 12 according to the "Double antigen sandwich assay method" described above, respectively, and the results are shown in Table 5 below.

**Table 5**

| **Reagent combination** | **Combination 1** | **Combination 11** | **Combination 12** |
|---|---|---|---|
| Negative Sample 1 (COI) | 0.11 | 0.15 | 0.12 |
| Negative Sample 2 (COI) | 0.16 | 0.21 | 0.14 |
| Positive Sample 1 (COI) | 2.78 | 3.24 | 3.06 |
| Positive Sample 2 (COI) | 13.83 | 16.77 | 15.23 |
| Positive Sample 3 (COI) | 85.50 | 74.43 | 71.59 |
| Negative coincidence rate (500 samples) | 100% | 100% | 100% |
| Positive coincidence rate (100 samples) | 100% | 100% | 100% |

As can be seen from Table 5, when using the antigenic protein fused in the order of Core+NS3+NS4+NS5, NS3+NS4+core+NS5 or NS3+core+NS4+NS5, both of the negative coincidence rate and the positive coincidence rate of the samples can reach 100%.

### Example 6

In order to investigate the HCV detection effect when using mutants of the enzyme, the following reagent combinations were prepared:
Combination 1: the *E. coli* expression system described above was used, a Core+NS3+NS4+NS5 chimeric protein was used to as an antigenic protein fuse with wild-type alkaline phosphatase to prepare the second reagent Rb, where in the fusion protein, wild-type alkaline phosphatase was fused at N-terminus of the antigen protein, and the Combination 1 was prepared according to "Reagent preparation method" above.
Combination 13: mutant alkaline phosphatase (GeneBank:M29670.1 (https://www.ncbi.nlm.nih.gov/)) was used, the rest was the same as combination 1.

Next, the 500 HCV negative samples and the 100 positive samples of Example 1 were tested using combinations 1 and 13 according to the "Double antigen sandwich assay method" described above, respectively, and the results are shown in Table 6.

**Table 6**

| **Reagent combination** | **Combination 1** | **Combination 13** |
|---|---|---|
| Negative Sample 1 (COI) | 0.11 | 0.13 |
| Negative Sample 2 (COI) | 0.16 | 0.15 |
| Positive Sample 1 (COI) | 2.78 | 4.56 |
| Positive Sample 2 (COI) | 13.83 | 24.11 |
| Positive Sample 3 (COI) | 85.50 | 150.73 |
| Negative coincidence rate (500 samples) | 100% | 100% |
| Positive coincidence rate (100 samples) | 100% | 100% |

It can be seen from Table 6 that when using wild-type alkaline phosphatase or mutant alkaline phosphatase, both of the negative coincidence rate and positive coincidence rate of the samples can reach 100%. In addition, detection discrimination is better when using mutant alkaline phosphatase compared to wild-type alkaline phosphatase.

### Example 7

In order to investigate the influence of different expression technologies on the HCV detection effect in the embodiments of the present application, a fusion protein of alkaline phosphatase and Core+NS3+NS4+NS5 was further prepared by yeast expression system, and combination 14 was prepared according to "Reagent preparation method".

The specific method of producing a fusion protein by yeast expression system was as follows:
Step 1: confirmation of the gene of interest. The nucleotide sequence GGA GGA GGA GGA TCA GGA GGA GGA GGA TCA GGA GGA GGA GGA TCA that capable of encoding a linker short peptide was added between the gene sequences of two proteins to be fused, and the corresponding amino acid sequence thereof was protein sequence (Gly₄Ser)₃, and a His tag was added to the C-terminus of the fusion protein for purification, the sequence of the gene of interest was preliminarily determined. Then, through codon optimization, the final sequence of the gene of interest suitable for *Pichia pastoris* expression strain *Pichia pastoris* yeast (X-33) was determined. In the embodiments of the present application, the number of amino acids in the linker short peptide was greater than 8, especially greater than 10.
Step 2: vector construction. A suitable enzyme restriction site was selected and the gene of interest was integrated into a suitable expression vector pPICZαA.
Step 3: transformation and screening of positive cloned strains. The expression vector containing the gene of interest was transformed into *Pichia pastoris* cells by electrotransformation. According to the reporter gene on the expression vector, an appropriate antibiotic was added to screen out positive strains.
Step 4: Expression and purification of the protein.

Next, the 500 HCV negative samples and the 100 positive samples of Example 1 were tested using combinations 1 and 14 according to the "Double antigen sandwich assay method" described above, respectively, and the results are shown in Table 7.

**Table 7**

| **Reagent combination** | **Combination 1** | **Combination 14** |
|---|---|---|
| Negative Sample 1 (COI) | 0.11 | 0.13 |
| Negative Sample 2 (COI) | 0.16 | 0.15 |
| Positive Sample 1 (COI) | 2.78 | 2.34 |
| Positive Sample 2 (COI) | 13.83 | 9.56 |
| Positive Sample 3 (COI) | 85.50 | 67.19 |
| Negative coincidence rate (500 samples) | 100% | 99.6% |
| Positive coincidence rate (100 samples) | 100% | 100% |

As can be seen from Table 7, in terms of signal-to-noise ratio, combination 1 of fusion protein prepared using the *E. coli* expression system is better than combination 14 of fusion protein prepared using the yeast expression system. In addition, the negative coincidence rate (100%) of combination 1 using the *E. coli* expression system is higher than that of combination 14 of fusion protein prepared using the yeast expression system (99.6%).

## Claims

1. A kit for detecting an HCV antibody comprising:
a fusion protein of an enzyme for labelling and an HCV antigenic protein,
wherein the antigenic protein contains one or more of HCV core antigen, HCV NS3 antigen, HCV NS4 antigen and HCV NS5 antigen, or a fusion antigen of two or more of the above-mentioned antigens; and
the enzyme for labelling is alkaline phosphatase or horseradish peroxidase.

2. The kit of claim 1, wherein the HCV antigenic protein is a fusion antigen containing HCV core antigen, HCV NS3 antigen, HCV NS4 antigen and HCV NS5 antigen.

3. The kit of claim 2, wherein the HCV antigenic protein contains, in the order from N-terminus to C-terminus, HCV core antigen, HCV NS3 antigen, HCV NS4 antigen and HCV NS5 antigen.

4. The kit of any one of claims 1 to 3, wherein the enzyme for labelling is fused at N-terminus or C-terminus of the antigenic protein.

5. The kit of claim 1, wherein the alkaline phosphatase is fused at N-terminus or C-terminus of the antigenic protein; or the horseradish peroxidase is fused at N-terminus or C-terminus of the antigenic protein.

6. The kit of claim 5, wherein the fusion protein contains, in the order from N-terminus to C-terminus, alkaline phosphatase, HCV core antigen, HCV NS3 antigen, HCV NS4 antigen and HCV NS5 antigen;
or, the fusion protein contains, in the order from N-terminus to C-terminus, HCV core antigen, HCV NS3 antigen, HCV NS4 antigen, HCV NS5 antigen and alkaline phosphatase;
or, the fusion protein contains, in the order from N-terminus to C-terminus, horseradish peroxidase, HCV core antigen, HCV NS3 antigen, HCV NS4 antigen and HCV NS5 antigen;
or, the fusion protein contains, in the order from N-terminus to C-terminus, HCV core antigen, HCV NS3 antigen, HCV NS4 antigen, HCV NS5 antigen and horseradish peroxidase.

7. The kit of claim 1, wherein the fusion protein of the enzyme for labelling and the HCV antigenic protein contains at least one of a fusion protein of the enzyme for labelling and HCV core antigen, a fusion protein of the enzyme for labelling and HCV NS3 antigen, a fusion protein of the enzyme for labelling and HCV NS4 antigen and a fusion protein of the enzyme for labelling and HCV NS5 antigen.

8. The kit of claim 7, wherein the fusion protein of the enzyme for labelling and the HCV antigenic protein contains the fusion protein of the enzyme for labelling and HCV core antigen, the fusion protein of the enzyme for labelling and HCV NS3 antigen, the fusion protein of the enzyme for labelling and HCV NS4 antigen and the fusion protein of the enzyme for labelling and HCV NS5 antigen.

9. The kit of any one of claims 1 to 8, further comprising:
a solid phase support coated with an HCV antigenic protein, wherein the HCV antigenic protein coated on the solid phase support contains one or more of HCV core antigen, HCV NS3 antigen, HCV NS4 antigen and HCV NS5 antigen, or a fusion antigen of two or more of the above-mentioned antigens, and the HCV antigenic protein coated on the solid phase support and the HCV antigenic protein in the fusion protein can bind to a same HCV antibody in a sample; or
a solid phase support coated with an anti-human IgG antibody and an anti-human IgM antibody.

10. The kit of claim 2 or 3 or 8, further comprising:
a solid phase support coated with a fusion antigen containing HCV core antigen, HCV NS3 antigen, HCV NS4 antigen, and HCV NS5 antigen; or
a solid phase support coated with HCV core antigen, a solid phase support coated with HCV NS3 antigen, a solid phase support coated with HCV NS4 antigen, and a solid phase support coated with HCV NS5 antigen.

11. The kit of any one of claims 1 to 10, further comprising a blocking agent; preferably, the blocking agent comprises one or more components selected from the group consisting of: skimmed milk powder, BSA, gelatin, serum, casein, ovalbumin, animal IgG and surfactant; and/or
further comprising a reducing agent; preferably, the reducing agent comprises one or more components selected from the group consisting of: DTT and β-mercaptoethanol; and/or
further comprising a reaction substrate for the enzyme for labelling, preferably the reaction substrate is 3-(2-spiroadamantane)-4-methoxy-4-(3-phosphoryloxy)-phenyl-1,2-dioxetane.

12. The kit of any one of claims 1 to 11, wherein the fusion protein is produced via prokaryotic expression technology.

13. A method for detecting an HCV antibody produced after infection with HCV in a sample, comprising:
mixing the sample and a solid phase support coated with a first ligand which is HCV antigenic protein, or anti-human IgG antibody and anti-human IgM antibody, so that the first ligand coated on the solid phase support is sufficiently bound to the HCV antibody in the sample;
washing the above mixture to remove unbound substances;
adding an enzyme label conjugate with a second ligand to the above washed mixture for mixing, in which the enzyme label conjugate with a second ligand is the fusion protein of an enzyme for labelling and an HCV antigenic protein defined in claim 1 and the HCV antigenic protein is the second ligand, so that the second ligand in the enzyme label conjugate binds to the HCV antibody bound on the solid phase support to form a sandwich complex;
washing the above sandwich complex to remove unbound substances;
adding a chemiluminescent substrate to the above washed sandwich complex, so that the above washed sandwich complex reacts with the chemiluminescent substrate, and detecting a number of photons generated by the reaction to obtain a chemiluminescent signal value of the sample.

## Patentansprüche

1. Ein Kit zum Nachweis eines HCV-Antikörpers,
das Folgendes umfasst:ein Fusionsprotein aus einem Markierungsenzym und einem HCV-Antigenprotein, wobei das Antigenprotein eines oder mehrere der folgenden Antigene enthält: HCV-Core-Antigen, HCV-NS3-Antigen, HCV-NS4-Antigen und HCV-NS5-Antigen, oder ein Fusionsantigen aus zwei oder mehr der oben genannten Antigene; und bei dem Markierungsenzym handelt es sich um eine alkalische Phosphatase oder Meerrettichperoxidase.

2. Kit nach Anspruch 1, wobei das HCV-Antigenprotein ein Fusionsantigen ist, das HCV-Core-Antigen, HCV-NS3-Antigen, HCV-NS4-Antigen und HCV-NS5-Antigen enthält.

3. Kit nach Anspruch 2, wobei das HCV-Antigenprotein, in der Reihenfolge vom N-Terminus zum C-Terminus, HCV-Core-Antigen, HCV-NS3-Antigen, HCV-NS4-Antigen und HCV-NS5-Antigen enthält.

4. Kit nach einem der Ansprüche 1 - 3, wobei das Markierungsenzym am N-Terminus oder C-Terminus des Antigenproteins fusioniert ist.

5. Kit nach Anspruch 1, wobei die alkalische Phosphatase am N-Terminus oder C-Terminus des Antigenproteins fusioniert ist; oder die Meerrettichperoxidase am N-Terminus oder C Terminus des Antigenproteins fusioniert ist.

6. Kit nach Anspruch 5, wobei das Fusionsprotein, in der Reihenfolge vom N-Terminus zum C-Terminus, alkalische Phosphatase, HCV-Core-Antigen, HCV-NS3-Antigen, HCV-NS4-Antigen und HCV-NS5-Antigen enthält;
oder das Fusionsprotein enthält, in der Reihenfolge vom N-Terminus zum C-Terminus, HCV-Core-Antigen, HCV-NS3-Antigen, HCV-NS4-Antigen, HCV-NS5-Antigen und alkalische
Phosphatase;
oder das Fusionsprotein enthält, in der Reihenfolge vom N-Terminus zum C-Terminus, Meerrettichperoxidase, HCV-Core-Antigen, HCV-NS3-Antigen, HCV-NS4-Antigen und HCV-NS5-Antigen;
oder das Fusionsprotein enthält, in der Reihenfolge vom N-Terminus zum C-Terminus, HCV- Core-Antigen, HCV-NS3-Antigen, HCV-NS4-Antigen, HCV-NS5-Antigen und Meerrettichperoxidase.

7. Kit nach Anspruch 1, wobei das Fusionsprotein aus dem Markierungsenzym und dem HCV-Antigenprotein mindestens eines der folgenden enthält: ein Fusionsprotein aus dem Markierungsenzym und HCV-Core-Antigen, ein Fusionsprotein aus dem Markierungsenzym und HCV-NS3-Antigen, ein Fusionsprotein aus dem Markierungsenzym und HCV-NS4-Antigen und ein Fusionsprotein aus dem Markierungsenzym und HCV-NS5-Antigen.

8. Kit nach Anspruch 7, wobei das Fusionsprotein aus dem Markierungsenzym und dem HCV-Antigenprotein das Fusionsprotein aus dem Markierungsenzym und HCV-Core-Antigen, das Fusionsprotein aus dem Markierungsenzym und HCV-NS3-Antigen, das Fusionsprotein aus dem Markierungsenzym und HCV-NS4-Antigen und das Fusionsprotein aus dem Markierungsenzym und HCV-NS5-Antigen enthält.

9. Das Kit nach einem der Ansprüche 1 -8 umfasst ferner:
eine Festphasenmatrix, beschichtet mit einem HCV-Antigenprotein, wobei das auf der Festphasenmatrix beschichtete HCV-Antigenprotein eines oder mehrere der folgenden enthält: HCV-Core-Antigen, HCV-NS3-Antigen, HCV-NS4-Antigen und HCV-NS5-Antigen, oder ein Fusionsantigen aus zwei oder mehr der oben genannten Antigene, und wobei das auf der Festphasenmatrix beschichtete HCV-Antigenprotein und das HCV-Antigenprotein im Fusionsprotein an denselben HCV-Antikörper in einer Probe binden können; oder
eine Festphasenmatrix, beschichtet mit einem Anti-Human-IgG-Antikörper und einem Anti-Human-IgM-Antikörper.

10. Das Kit nach Anspruch 2 oder 3 oder 8 umfasst ferner:
eine Festphasenmatrix, beschichtet mit einem Fusionsantigen, das HCV-Core-Antigen, HCV-NS3-Antigen, HCV-NS4-Antigen und HCV-NS5-Antigen enthält; oder
eine Festphasenmatrix, beschichtet mit HCV-Core-Antigen, eine Festphasenmatrix, beschichtet mit HCV-NS3-Antigen, eine Festphasenmatrix, beschichtet mit HCV-NS4-Antigen, und eine Festphasenmatrix, beschichtet mit HCV-NS5-Antigen.

11. Das Kit nach einem der Ansprüche 1 bis 10 umfasst ferner: ein Blockierungsmittel, das vorzugsweise eine oder mehrere Komponenten aus der Gruppe enthält, bestehend aus: Magermilchpulver, BSA, Gelatine, Serum, Casein, Ovalbumin, tierisches IgG und Tensid; und/oder
ein Reduktionsmittel, das vorzugsweise eine oder mehrere Komponenten aus der Gruppe bestehend aus DTT und β-Mercaptoethanol enthält, und/oder
ein Reaktionssubstrat für das Markierungsenzym, wobei es sich vorzugsweise um das Reaktionssubstrat 3-(2-Spiroadamantyl)-4-methoxy-4-(3-phosphoryloxy)-phenyl-1,2-dioxetan handelt.

12. Kit nach einem der Ansprüche 1 - 11, wobei das Fusionsprotein mittels prokaryotischer Expressionstechnologie hergestellt wird.

13. Verfahren zum Nachweis eines nach einer Infektion mit HCV gebildeten HCV-Antikörpers in einer Probe, Folgendes umfasst:
Mischen der Probe mit einer Festphasenmatrix, die mit einem ersten Liganden beschichtet ist, wobei es sich dabei um ein HCV-Antigenprotein oder ein Anti-human-IgG-Antikörper und Anti-human-IgM-Antikörper handelt, sodass der erste Ligand auf der Festphasenmatrix ausreichend an den HCV-Antikörper in der Probe bindet;
Waschen des oben genannten Gemischs zur Entfernung ungebundener Substanzen;
Zugabe eines Enzymmarkierungs-Konjugats mit einem zweiten Liganden zu dem gewaschenen Gemisch zum Mischen, wobei das Enzymmarkierungs-Konjugat mit einem zweiten Liganden das in Anspruch 1 definierte Fusionsprotein aus einem Markierungsenzym und einem HCV-Antigenprotein ist und das HCV-Antigenprotein der zweite Ligand ist, sodass der zweite Ligand im Enzymmarkierungs-Konjugat an den auf der Festphasenmatrix gebundenen HCV-Antikörper bindet, um einen Sandwichkomplex zu bilden;
Waschen des oben genannten Sandwichkomplexes zur Entfernung ungebundener Substanzen; Zugabe eines chemilumineszierenden Substrats zu dem gewaschenen Sandwichkomplex, sodass dieses mit dem chemilumineszierenden Substrat reagiert, und Nachweis der durch die Reaktion erzeugten Photonenanzahl, um einen Signalwert des chemilumineszierenden Substrats der Probe zu erhalten.

## Revendications

1. Kit de détection d'un anticorps contre le VHC comprenant :
une protéine de fusion d'une enzyme de marquage et d'une protéine antigénique du VHC,
où la protéine antigénique contient un ou plusieurs antigènes parmi l'antigène core du VHC, l'antigène NS3 du VHC, l'antigène NS4 du VHC et l'antigène NS5 du VHC, ou un antigène de fusion de deux ou plus des antigènes susmentionnés ; et
l'enzyme de marquage est une phosphatase alcaline ou une peroxydase de raifort.

2. Kit selon la revendication 1, dans lequel la protéine antigénique du VHC est un antigène de fusion contenant l'antigène core du VHC, l'antigène NS3 du VHC, l'antigène NS4 du VHC et l'antigène NS5 du VHC.

3. Kit selon la revendication 2, dans lequel la protéine antigénique du VHC contient, dans l'ordre de l'extrémité N-terminale vers l'extrémité C-terminale, l'antigène core du VHC, l'antigène NS3 du VHC, l'antigène NS4 du VHC et l'antigène NS5 du VHC.

4. Kit selon l'une quelconque des revendications 1 à 3, dans lequel l'enzyme de marquage est fusionnée au niveau de l'extrémité N-terminale ou de l'extrémité C-terminale de la protéine antigénique.

5. Kit selon la revendication 1, dans lequel la phosphatase alcaline est fusionnée au niveau de l'extrémité N-terminale ou de l'extrémité C-terminale de la protéine antigénique ; ou la peroxydase de raifort est fusionnée au niveau de l'extrémité N-terminale ou de l'extrémité C-terminale de la protéine antigénique.

6. Kit selon la revendication 5, dans lequel la protéine de fusion contient, dans l'ordre de l'extrémité N-terminale vers l'extrémité C-terminale, une phosphatase alcaline, l'antigène core du VHC, l'antigène NS3 du VHC, l'antigène NS4 du VHC et l'antigène NS5 du VHC ;
ou, la protéine de fusion contient, dans l'ordre de l'extrémité N-terminale vers l'extrémité C-terminale, l'antigène core du VHC, l'antigène NS3 du VHC, l'antigène NS4 du VHC, l'antigène NS5 du VHC et une phosphatase alcaline ;
ou, la protéine de fusion contient, dans l'ordre de l'extrémité N-terminale vers l'extrémité C-terminale, une peroxydase de raifort, l'antigène core du VHC, l'antigène NS3 du VHC, l'antigène NS4 du VHC et l'antigène NS5 du VHC ;
ou, la protéine de fusion contient, dans l'ordre de l'extrémité N-terminale vers l'extrémité C-terminale, l'antigène core du VHC, l'antigène NS3 du VHC, l'antigène NS4 du VHC, l'antigène NS5 du VHC et une peroxydase de raifort.

7. Kit selon la revendication 1, dans lequel la protéine de fusion de l'enzyme de marquage et de la protéine antigénique du VHC contient au moins l'un d'une protéine de fusion de l'enzyme de marquage et de l'antigène core du VHC, d'une protéine de fusion de l'enzyme de marquage et de l'antigène NS3 du VHC, d'une protéine de fusion de l'enzyme de marquage et de l'antigène NS4 du VHC et d'une protéine de fusion de l'enzyme de marquage et de l'antigène NS5 du VHC.

8. Kit selon la revendication 7, dans lequel la protéine de fusion de l'enzyme de marquage et de la protéine antigénique du VHC contient la protéine de fusion de l'enzyme de marquage et de l'antigène core du VHC, la protéine de fusion de l'enzyme de marquage et de l'antigène NS3 du VHC, la protéine de fusion de l'enzyme de marquage et de l'antigène NS4 du VHC et la protéine de fusion de l'enzyme de marquage et de l'antigène NS5 du VHC.

9. Kit selon l'une quelconque des revendications 1 à 8, comprenant en outre :
un support en phase solide revêtu d'une protéine antigénique du VHC, où la protéine antigénique du VHC appliquée sur le support en phase solide contient un ou plusieurs antigènes parmi l'antigène core du VHC, l'antigène NS3 du VHC, l'antigène NS4 du VHC et l'antigène NS5 du VHC, ou un antigène de fusion de deux ou plus des antigènes susmentionnés, et la protéine antigénique du VHC appliquée sur le support en phase solide et la protéine antigénique du VHC dans la protéine de fusion peuvent se lier sur un même anticorps contre le VHC dans un échantillon ; ou
un support en phase solide revêtu d'un anticorps anti-IgG humaine et d'un anticorps anti-IgM humaine.

10. Kit selon la revendication 2 ou 3 ou 8, comprenant en outre :
un support en phase solide revêtu d'un antigène de fusion contenant l'antigène core du VHC, l'antigène NS3 du VHC, l'antigène NS4 du VHC et l'antigène NS5 du VHC ; ou
un support en phase solide revêtu de l'antigène core du VHC, un support en phase solide revêtu de l'antigène NS3 du VHC, un support en phase solide revêtu de l'antigène NS4 du VHC et un support en phase solide revêtu de l'antigène NS5 du VHC.

11. Kit selon l'une quelconque des revendications 1 à 10, comprenant en outre un agent bloquant ; de préférence, l'agent bloquant comprend un ou plusieurs composants sélectionnés parmi le groupe constitué : de poudre de lait écrémé, de BSA, de gélatine, de sérum, de caséine, d'ovalbumine, d'IgG animale et d'un tensioactif ; et/ou
comprenant en outre un agent réducteur ; de préférence l'agent réducteur comprend un ou plusieurs composants sélectionnés parmi le groupe constitué : de DTT et de β-mercaptoéthanol ; et/ou
comprenant en outre un substrat de réaction pour l'enzyme de marquage, de préférence le substrat de réaction est le 3-(2-spiroadamantane)-4-méthoxy-4-(3-phosphoryloxy)-phényl-1,2-dioxétane.

12. Kit selon l'une quelconque des revendications 1 à 11, dans lequel la protéine de fusion est produite via une technologie d'expression procaryotique.

13. Procédé de détection d'un anticorps contre le VHC produit à la suite d'une infection par le VHC dans un échantillon, comprenant les étapes consistant à :
mélanger l'échantillon et un support en phase solide revêtu avec un premier ligand lequel est une protéine antigénique du VHC, ou un anticorps anti-IgG humaine et un anticorps anti-IgM humaine, de sorte que le premier ligand appliqué sur le support en phase solide est suffisamment lié à l'anticorps contre le VHC dans l'échantillon ;
laver le mélange susmentionné pour éliminer les substances non liées ;
ajouter un conjugué d'enzyme de marquage avec un second ligand au mélange lavé susmentionné pour mélange, où le conjugué d'enzyme de marquage avec un second ligand est la protéine de fusion d'une enzyme de marquage et d'une protéine antigénique du VHC définie à la revendication 1 et la protéine antigénique du VHC est le second ligand, de sorte que le second ligand dans le conjugué d'enzyme de marquage se lie à l'anticorps contre le VHC lié sur le support en phase solide pour former un complexe en sandwich ;
laver le complexe en sandwich susmentionné pour éliminer les substances non liées ;
ajouter un substrat chimiluminescent au complexe en sandwich lavé susmentionné, de sorte que le complexe en sandwich lavé susmentionné réagit avec le substrat chimiluminescent, et détecter un nombre de photons générés par la réaction pour obtenir une valeur de signal chimiluminescent de l'échantillon.
